# EUROPEAN PATENT SPECIFICATION

(11) **EP 1 556 436 B1**
(45) Date of publication and mention of the grant of the patent: **21.06.2006**
(21) Application number: 03809726.7
(22) Date of filing: 20.10.2003
(51) Int. Cl.: C08G 73/02, C07C 235/10, C07C 271/20

(54) **POLYAMINE DERIVATIVES, A PROCESS TO MAKE THEM, AND THEIR USE**
POLYAMINDERIVATE, HERSTELLUNGSVERFAHREN DAFÜR UND VERWENDUNG DAVON
DERIVES DE POLYAMINE, PROCEDE DE FABRICATION ET UTILISATION DE CES DERNIERS

(30) Priority: 29.10.2002 EP 02079516
(43) Date of publication of application: 27.07.2005
(73) Proprietor: Akzo Nobel N.V., 6824 BM Arnhem (NL)
(72) Inventor: PARDOEN, Johannes, Adrianus, NL-4661 MC Halsteren (NL); BRINKHUIS, Richard, Hendrikus, Gerrit, NL-8011 JX Zwolle (NL); VENDERBOSCH, Rudolf, Anthonius, Maria, NL-6922 JL Duiven (NL)
(74) Representative: Jönsson, Christer
(86) International application number: PCT/EP2003/011647
(87) International publication number: WO 2004/039865

(56) References cited:
- EP-A- 0 358 358
- WO-A-02/072639
- US-B1- 6 197 877
- PATENT ABSTRACTS OF JAPAN vol. 1997, no. 10, 31 October 1997 (1997-10-31) & JP 9 157374 A (DAICEL CHEM IND LTD), 17 June 1997 (1997-06-17)

## Description

The invention relates to a process to make polyamine derivatives with at least one anchoring group and at least one matrix-compatible moiety, the polyamine derivatives so obtainable, and the use of such polyamine derivatives in conventional applications, such as pigment dispersants in inks, wetting agents in coating compositions, and transfer resins in pigment processing operations.

EP-A-0 358 358 discloses the use of specific amine derivatives as pigment dispersing agents. However, such conventional polyester based pigment dispersants (see also WO 98/19784) often lead to crystallisation problems, particularly when they are used in acryloyl-functional acrylic resin-based compositions. Further, some conventional pigment dispersants contain salt bridges, which is undesired.

Patent Abstracts of Japan Vol. 1997, No. 10, 31 Oct 1997, abstract of JP 09-157374, discloses pigment dispersant obtained by reacting an aromatic-group-modified polyamine compound component with a polyester compound component having lactone-derived units.

There is a need for alternative pigment dispersing agents in the art, especially for applications such as printing inks, varnishes, and coating compositions that can be cured chemically and/or photochemically. These alternative agents preferably have better dispersing properties than the conventional pigment dispersants and preferably are suitable in virtually all conventional applications, without any further processing steps, such as the re-heating of the formulation to (re)dissolve any precipitate, being required. Finally, the alternative agents should preferably be substantially free of salt bridges.

Acryloyl-functional resin-based coating compositions have become more popular, since they are very useful in coating compositions which are cured by at least a UV curing step. There is a specific need for alternative pigment dispersing agents for UV cure applications, such as UV curable printing inks, varnishes, and coating compositions.

The present invention relates to alternative agents that are of use as pigment dispersant. In a preferred embodiment it relates to specific polyamine derivatives containing matrix-compatible moieties.

Surprisingly, we have found a process to make polyamine derivatives which are very versatile and typically will outperform the products of EP-A-0 358 358 and WO 98/19784 in pigment dispersing operations, particularly since the anchoring moiety of the polyamine derivatives has improved interaction with pigments, while the matrix-compatible moiety can be tailored to ensure full compatibility of the derivatives and the matrix in which they are used, particularly in combination with acryloyl-functional resins. Accordingly, we claim the process to make these polyamine derivatives, intermediates of the process, the products obtainable, and their use.

The process according to the invention is characterised in that one or more polyamines, each with one or more -NH₂ functions and one or more second amine functions, said second amine functions having a lower lactone reactivity than said -NH₂ functions, is reacted in a first step with one or more lactones, hydroxyacids, cyclic carbonates, or mixtures thereof, to form a polyamine-derived compound with amide and/or urethane groups, which polyamine-derived compound is reacted in a second step with one or more at least bifunctional amine-specific reagents to form an intermediate optionally comprising ester and/or carbonate groups, wherein in the second step optionally an additional amine modifier of formula III)

(Y)ₓ(Y")_{y}(Y')_{z}NH_{3-x-y-z} III)

wherein x is an integer of 0, 1 or 2, y is an integer of 0, 1 or 2, z is an integer of 0 or 1, wherein x+y is 1 or 2, x+y+z is 1 or 2, Y represents an (anchoring) moiety with affinity for a pigment surface or substrate, Y" represents a (stabilising) moiety with affinity for the matrix, and Y' represents a further group that is neither an anchoring moiety nor a stabilising moiety, is co-reacted, and in the intermediate at least two polyamine residues, or if a modifier is co-reacted, at least one polyamine residue and at least one optional amine modifier residue, are linked by said bifunctional amine-specific reagent.

Affinity for the matrix as used herein means that a mixture of the matrix and the moiety with affinity for the matrix as such gives a homogeneous solution. In thermodynamic terms this means that the interaction between the matrix and the moiety with affinity for the matrix should be better than theta (for theta conditions see "Introduction to Polymers", 2^{nd} edition, p. 162, by R.J. Young and P. A. Lovell). Affinity of a compound for the pigment surface or the substrate as used herein is determined by dissolving the compound in the matrix in a known concentration. This solution is subsequently mixed with 5 wt% of pigment particles or substrate particles (the substrate particles having a size about 1 mm), respectively, at atmospheric conditions. After 24 hours of mixing, the concentration of the compound in the matrix is determined again. If the concentration of the compound has decreased the compound has an affinity for the pigment or substrate, respectively.

In a preferred embodiment the process according to the invention is characterised in that one or more polyamines, each with one or more -NH₂ functions and one or more second amine functions, said second amine functions having a lower lactone reactivity than said -NH₂ functions and wherein each second amine function, independently, can be primary or secondary, is reacted in a first step with one or more lactones, cyclic carbonates, hydroxyacids, or mixtures thereof, to form a polyamine-derived compound comprising unreacted second amine function, amide and/or urethane groups, optionally ester and/or carbonate groups, and one or more -OH groups, which compound is reacted in a second step with an at least bifunctional amine-specific reagent to form an intermediate comprising at least two polyamine residues with amide and/or urethane groups and optionally ester and/or carbonate groups that are linked by said amine-specific reagent residue.

If so desired, any conventional catalyst may be used in either the first or the second step. The molecular ratio in which the polyamines are reacted with said lactones, cyclic carbonates, hydroxyacids, or mixtures thereof can vary over a wide range. Preferably, on average at least 0.1, more preferably at least 0.5, most preferably at least 1 lactone, cyclic carbonate and/or hydroxyacid molecule is reacted per -NH₂ function in the reaction mixture of the first step. Similarly, preferably on average at most 10, more preferably at most 5, even more preferably at most 3, still more preferably at most 2, and most preferably at most 1.5 lactone, cyclic carbonate and/or hydroxyacid molecule is reacted per -NH₂ function.

The linked polyamine residue(s) with amide and/or urethane groups and optional ester and/or carbonate groups were found to form an "anchoring group" with very good interaction (affinity) with pigments and plastic surfaces. Depending on the molar ratio of the reactants in the first step and the nature of the at least bifunctional amine-specific reagent, it is possible to tailor the anchoring group to optimise this interaction. Optionally, but less preferred, the process comprises an intermediate step wherein the -OH groups, or parts of the unreacted amine groups, are reacted before the step wherein the polyamine residues are linked with the at least bifunctional amine-specific reagent. If this intermediate step is performed, then care is to be taken that the second amine functions survive the reaction step, in order to allow the linking to occur.

The polyamines used in accordance with the invention are substituted or unsubstituted, linear or branched, hydrocarbons containing one or more -NH₂ groups and one or more second amine functions with a lower lactone reactivity than said -NH₂ functions. Although less preferred, these polyamines may also possess other less reactive groups, e.g. hydroxyl groups. Further, although also less preferred, the polyamines may only contain amine groups with a comparable reactivity which in a first step are reacted with less than 1 equivalent of the lactone, cyclic carbonate or hydroxyacid compound. To establish whether or not the amine groups of the polyamine have the necessary difference in reactivity towards lactones and/or cyclic carbonates, one can start from common general knowledge with respect to the reactivity of amines, including the knowledge that, in such reactions, primary amines are more reactive than secondary amines, and primary amines on secondary carbon atoms are more reactive than primary amine groups on tertiary carbon atoms. Helpful is the simple test wherein 1 mole of ε-caprolactone is reacted with a mixture comprising 1 mole of a low-molecular weight mono-amine analogue with said -NH₂ group and 1 mole of a low-molecular weight mono-amine analogue with said second amine function. The reaction can be performed with or without solvents at a temperature where reactivity is observed which can be monitored with; e.g., a titration on amines. Suitably the reaction is conducted at 100°C for a period of 16 hours. Low-molecular weight compounds are suitably compounds with a molecular weight of from 50 to 150 Dalton. In the test but, if this can be analysed, preferably in the reaction of the polyamine, at least 60%, on a molar basis, of the lactone, hydroxy acid and/or cyclic carbonate reacts with the more reactive -NH₂ functions. More preferably, at least 70%, on a molar basis, of the lactone, hydroxyacid and/or cyclic carbonate reacts with the -NH₂ function, and most preferably, at least 80%, on a molar basis, of the lactone, hydroxyacid and/or cyclic carbonate reacts with the more reactive -NH₂ functions.

Preferred polyamines are compounds of formula I), 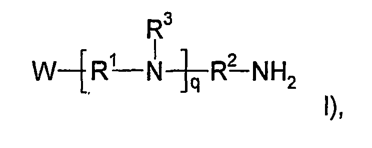 wherein q is an integer from 1 to 10, preferably 1 to 5, more preferably 1 or 2, R¹ and R², independently, represent alkylene groups with from 1 to 10 carbon atoms, preferably from 1 to 6 carbon atoms, each of R³ being independently selected from hydrogen, hydroxyalkyl, alkylamine, polyalkylamine, and polyalkylpolyamine, and W is hydroxy or amine. Preferably, R¹ and R² are the same. Examples of products according to formula I) include: diethylene triamine (q=1), triethylene tetramine (q=2), tetraethylene pentamine (q=3), triethylene tetramine (q=1, R³ is ethylamine), dipropylene triamine (q=1), dihexylene triamine (q=1), and compounds of the general branched structure 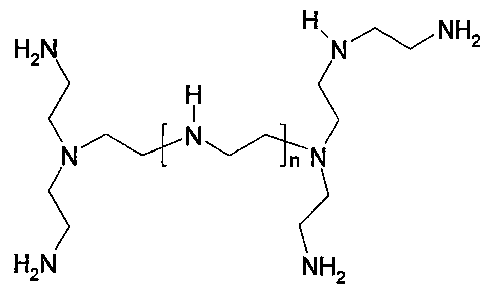 prepared from aziridine and known as polyethylene imine and commercially available under the tradename Epomin® SP with Mw's up to 10,000 or similar, e.g. compounds having propylene rather than ethylene moieties, or compounds with more or less primary, secondary, or tertiary amine groups. The compounds can also comprise a combination of ethylene, propylene and/or hexylene units.

The lactone, hydroxyacid and/or cyclic carbonate can be selected from any conventional lactone, hydroxy acid and/or cyclic carbonate, including, but not limited to, butyrolactones, valerolactones, caprolactones, ethylene carbonate, propylene carbonate, glycerol carbonate, hydroxy stearic acids, hydroxybutyric acids, and hydroxycaproic acid. Preferably, a conventional lactone is selected. The lactones can be of the beta-, gamma-, delta- and/or epsilon-type. In view of their stability and availability, gamma-, delta-, and epsilon-lactones are most preferred.

The product of the first step is reacted with an at least bifunctional amine-specific reagent. By amine-specific is meant that if the polyamine-derived compound comprises both the second amine function and -OH groups, then at least 60%, on a molar basis, of the amine-specific reagent groups will react with the second amine function. Preferably, at least 80%, most preferably at least 90%, both on a molar basis, of the amine-specific reagent groups will react with the second amine function of the product of the first step. Preferably, the specificity of the reaction is measured by analysing the reaction product of the process according to the invention. However, if this is not feasible, then a simple test method can be used wherein a mixture comprising an equal molar amount of a low-molecular weight mono-OH-functional analogue and a low-molecular weight analogue compound with just one second amine function is reacted in a suitable solvent at a temperature of 65°C, optionally in the presence of a solvent, until at least 80%, preferably at least 90% of the amine has reacted with the amine-specific reagent, after which the specificity of the reaction can be determined using conventional techniques.

The amine-specific reagent is typically used in an amount wherein the number of amine-reactive groups corresponds to from 0.1 to 10 times the number of second amine function groups of the product of the first step. In a preferred embodiment, the amine-specific reagent is used in an amount wherein the number (in equivalents) of amine-reactive groups corresponds to from 1 to 10 times the number (in equivalents) of second amine function groups of the polyamine-derived compound. In particular, if the amine-specific reagent comprises three or more reactive groups, up to 10 times equivalent amine-reactive groups on 1 equivalent second amine function group will still provide sufficient crosslinking in the resulting intermediate of the reaction between the amine-specific reagent and the product of the first step. By sufficient crosslinking is meant that, on average, at least two amine-functional compounds are linked to an amine-specific reagent. Preferred amine-specific reagents include isocyanates, anhydrides, acid chlorides, epoxy compounds, maleates, fumarates, citraconic esters, itaconic esters, and (meth)acrylates. More preferably, the amine-specific reagent is a conventional di- or poly-isocyanate, including compounds such as hexylene-1,6-diisocyanate, isophorone diisocyanate, toluene diisocyanate, and oligomeric or polymeric diphenyl methane diisocyanate (MDI). It is to be understood that if the amine-specific reagent comprises more than two amine-reactive groups, then these additional groups may or may not have reacted with amine functions of the same or other polyamine-derived compound molecules.

The intermediate from the second reaction step comprises -OH groups, which are reacted in a third step of a preferred process with compounds that introduce a "matrix-compatible moiety". By matrix-compatible is meant that said moiety has affinity for the continuous medium in which the final polyamine derivative is to be used as dispersant, wetting agent, or the like. Preferably, the polyamine derivative is used in coating compositions and printing inks, most preferably in acryloyl-functional resin-containing compositions. Non-aqueous coating compositions and printing inks are preferred. The matrix-compatible moiety has a molecular weight of more than 250, preferably more than 500, more preferably more than 800, even more preferably more than 1,200 and, most preferably, more than 2,000 Dalton, with the molecular weight preferably being below 20,000, more preferably below 10,000 Dalton. If the polyamine derivative is to be used in aqueous compositions, e.g. as a paint dispersant in water-borne coating compositions, then the third step preferably comprises an alkoxylation process. Said alkoxylation process can comprise one or more ethoxylation and/or propoxylation steps, in any order or sequence.

If the polyamine derivative is to be used in non-aqueous compositions, then at least part of the OH groups of the intermediate is preferably reacted with an epoxide, lactone, cyclic carbonate, (hydroxy) acid, ester, anhydride, etc., to substitute a linear or branched, substituted or unsubstituted, preferably unsubstituted, C₄-C₃₀ alkyl, polyester, polyether, polyetherester and/or polyesterether group for the proton.

It should be noted that the polyamine derivative can be modified at any time during the process, provided that the final product resulting from the process bears at least one anchoring group and at least one matrix-compatible moiety. Two examples of preferred modifications are the following:
1.) in the second step of the process, at least one additional amine modifier is introduced of formula III),

   (Y)ₓ(Y")_{y}(Y')_{z}NH_{3-x-y-z} III)

   wherein x is an integer of 0, 1 or 2, y is an integer of 0, 1 or 2, z is an integer of 0 or 1, wherein x+y is 1 or 2, and x+y+z is 1 or 2, Y represents an (anchoring) moiety with affinity for a pigment surface or substrate (such as a plastic article), Y" represents a (stabilising) moiety with affinity for the matrix, and Y' represents a further group that is neither an anchoring moiety nor a stabilising moiety. Group Y' may be any group known by the person skilled in the art, but is preferably selected from the group of branched and unbranched alkyl and cycloalkyl groups, preferably C₁-C₁₀ alkyl and cycloalkyl groups, more preferably C₃-C₁₀ alkyl and cycloalkyl groups. Preferably, group Y" is selected from the group of linear or branched, substituted or unsubstituted, preferably unsubstituted, C₄-C₃₀ alkyl, polyester, polyether, polyetherester, and polyesterether. Preferably, group Y is an aliphatic group with from 2 to 10 carbon atoms containing at least one tertiary amino group or a heterocyclic group containing at least one basic ring nitrogen atom and where the heterocyclic group may be attached to the NH group of formula III) by an alkylene group containing up to 10 carbon atoms. Preferred heterocyclic groups are optionally substituted triazole, pyrimidine, imidazole, pyridine, morpholine, pyrrolidine, piperazine, benzimidazole, benzthiazole and/or triazine. Substituents may be C₁₋₆ and especially C₁₋₄-alkyl or alkoxy or amino. As noted hereinbefore, Y may be attached to the NH group via an alkylene group containing 2 to 10 carbon atoms, preferably it is a C₂₋₈-alkylene, more preferably a C₂₋₅-alkylene, and even more preferably a C₂₋₄-alkylene group. Y may also be attached to the NH group via a polyether group containing the same number of carbon atoms as the alkylene group. Examples of still more preferred products of formula III) are: nitrogen-substituted alkanediamines with an NH₂ group and a secondary or tertiary amine function, 1-(2-aminoethyl)-piperazine, 2-(1-pyrrolidyl)-ethylamine, 4-amino-2-methoxypyrimidine, products of formula IV), 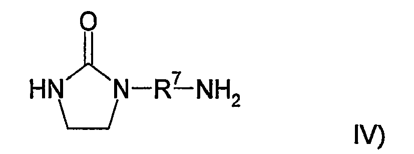 wherein R⁷ is an ethylene group, and Jeffamines®. Most preferably, the compounds are selected from the group consisting of N, N-dimethyl-1,3-propanediamine, 4-(2-aminoethyl)-pyridine, 2-amino-6-methoxybenzothiazole, 4-(aminomethyl)-pyridine, N,N-diallylmelamine, 3-amino-1,2,4-triazole, 1-(3-aminopropyl)-imidazole, 3-mercapto-1,2,4-triazole, monofunctional Jeffamines®, and products of formula IV). The additional amine was found to be incorporated into the anchoring group, allowing further tailoring. The amount of at least bifunctional amine-specific reagent in the second step is to be adjusted such that sufficient bifunctional amine-specific reagent is present to also react with the additional amine.
2.) At least one (additional) monofunctional amine-specific reagent of formula V-A is present in the second step of the process, wherein V is selected from the group consisting of linear or branched, substituted or unsubstituted, preferably unsubstituted, C₄-C₃₀ alkyl, polyester, polyether, polyetherester, and polyesterether, and A is an amine-specific reactive group as defined for the second step of the process. The additional monofunctional amine-specific reagent was found to be attached to the anchoring group, allowing further tailoring of the final polyamine derivative, to optimise it for use in various media, using varying pigments and/or plastic substrates. The amount of at least bifunctional amine-specific reagent in the second step may be adjusted such that the total amount of reactive groups of the at least bifunctional amine-specific reagent and the monofunctional amine-specific reagent is within the range of 1 to 10 times the number of second amine function groups of the reaction mixture.

In another preferred embodiment, the invention also relates to compounds obtainable by the above-identified process which comprise said anchoring and matrix-compatible moieties. Such compounds can be represented by formula II): 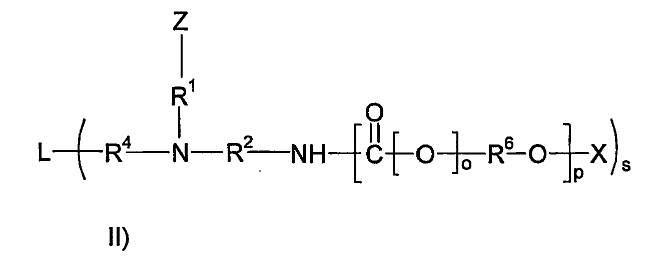 wherein each R⁴NR¹ZR²NH moiety is a residue of the polyamine used, each C(O)[O]R⁶O moiety is a residue of the lactone, hydroxyacid and/or cyclic carbonate used, and L is the residue of the at least bifunctional amine-specific reagent. R¹ and R² are as defined above for formula I. Depending whether or not a lactone, hydroxy acid or cyclic carbonate is used, each o will independently be 0, 0, and 1, respectively. The index p presents the average number of moiety C(O)[O]R⁶O per R⁴NR¹ZR²NH moiety and has a value ranging from 0.1 to 30, preferably from 0.1 to 10, more preferably 0.5 to 5, most preferably 1 to 3. The tail thus formed of moieties C(O)[O]R⁶O preferably is of a non-crystallising nature and further, preferably, is not completely formed of caprolactone derivatives. For the molecules derived from the above-mentioned process, each X preferably is hydrogen. However, if the optional intermediate modification step as presented above is taken or if the -OH group is reacted in later steps, then X is, wholly or partly, a substituted or unsubstituted, linear or branched, hydrocarbon group. The index s represents the number of products of the first reaction step that react with the amine-specific reagent and is an integer of 1 to 10. If s is 1, the amine-specific reagent L is further reacted with a compound of formula III). Preferably, s is 1 to 4, most preferably s is 1 or 2. R⁴ presents group R³ minus a proton. R³ is as defined above for formula I). Z- presents a group W'-[R¹-NR⁵]_{q-1}-, wherein W' is W as defined for formula I above or the reaction product of group W with lactone, hydroxyacid and/or cyclic carbonate, and each R⁵ independently is a group R³ or the reaction product of R³ with amine-specific reagent L.

The moiety of formula II), minus the groups X, was found to be a very suitable anchoring group for interaction with (affinity for) a variety of pigments and substrates. However, in order to make a good dispersant, the moiety is to be combined with at least one matrix-compatible moiety to form suitable dispersants, wetting agents, compatibilisers, and the like. Accordingly, each group X, independently, preferably is such a matrix-compatible moiety. Typically, this is achieved by reacting the product of formula II), wherein X is hydrogen with a suitable compound. Preferably, each of the groups X, independently, is a compound with a molecular weight of more than 250, preferably more than 500, more preferably more than 800, even more preferably more than 1,200 and, most preferably, more than 2,000 Dalton, and a molecular weight of preferably below 20,000, more preferably below 10,000 Dalton. In a preferred embodiment, the groups X are fully compatible with binder resins and/or the solvent of the composition in which the products according to the invention are used.

In a further preferred embodiment the groups X are selected such that the final compound is fully compatible with acryloyl-functional resins. By fully compatible is meant that the groups X as such are fully soluble in the matrix. Preferably, the groups X are, independently, selected from linear or branched, substituted or unsubstituted, preferably unsubstituted, C₄-C₃₀ alkyl, polyester, polyether, polyetherester or polyesterether groups. Preferably, the groups X are formed by reacting a compound of formula II), wherein X is hydrogen with appropriate epoxides, lactones, cyclic carbonates, hydroxy acids, and other suitable conventional reactants to form polyesters, e.g. via an alternating co-polymerisation, and/or polyethers. Alternatively, a linear or branched, substituted or unsubstituted, preferably unsubstituted, C₄-C₃₀ alkyl, polyester, polyether, polyetherester or polyesterether with OH reactive groups is reacted with compound II), with X is hydrogen. If so desired, conventional catalysts may be used in this step.

If the solvent of the composition in which the polyamine derivative is used is an organic solvent, it is most preferred that X comprises a polyester tail. If the solvent of the composition in which the polyamine derivative is used is an aqueous solvent, it is most preferred that X comprises a polyether tail.

Especially good polyamine derivatives are obtained when the -OH function of the polar moiety is reacted in an alternating co-polymerisation with hexahydrophthalic anhydride and Cardura® E10, to form a polyester function. Notwithstanding the above, if the compound of formula II as defined above comprises other moieties that are compatible with the solvent and/or binder resin of the composition wherein the compound of formula II is used, (part of) X may simply be a hydrogen atom. In this respect, further especially preferred polyamine derivatives are polyamine derivatives comprising one or more groups derived from reaction with an amine modifier of formula III) described above, wherein this group acts as a stabilising group in the composition in which the polyamine derivative is used. Compounds of formula III) that can provide a group that acts as a stabilising group in water include mono-functional Jeffamines®. Also, group Z of formula II) may act as a stabilising group in the composition in which the polyamine derivative is used.

The invention also relates to the use of the polyamine derivatives prepared by the process given above in coating compositions or printing ink formulations. Preferably, the polyamine derivatives are used in a coating composition or printing ink formulation that comprises acryloyl-functional resins. The polyamine derivatives are of particular use as pigment dispersants in pigmented coatings and/or printing inks. In a more preferred embodiment the polyamine derivatives are used in a coating or ink composition that is curable by actinic light irradiation, such as UV or IR irradiation. Most preferably, such compositions comprise acryloyl-functional resins. Other preferred uses of the polyamine derivatives are as a wetting agent or as adhesion promotion agent in a coating composition or as a transfer resin in pigment processing operations.

The invention is elucidated by the following examples.

### Example 1: Preparation of a pigment dispersant with polyester tails

### a) Preparation of a hydroxy amide-amine:

Into a 250 ml three-necked round-bottomed flask equipped with a mechanical stirrer, a N₂inlet, and a reflux condenser, 61.85 grams (0.60 mole) of diethylene triamine (ex Aldrich) and 136.85 grams (1.20 moles) of ε-caprolactone (ex Aldrich) were weighed. The reaction mixture was heated on an oil bath, at 140°C, for 10 h to complete the reaction. A polyamine-derived compound was obtained.

### b) Preparation of a tetrahydroxy-tetraamide-diurea

Into a 250 ml three-necked round-bottomed flask equipped with a mechanical stirrer, a N₂-inlet, and a reflux condenser, 96.90 grams (0.292 mole) of the polyamine-derived compound of Example 1a was weighed. The temperature was raised to 65°C and 24.50 grams (0.146 mole) of hexamethylene diisocyanate (ex Fluka) were added over a period of 45 minutes while keeping the reaction mixture at 65°C. The temperature was next raised to 100°C in order to complete the reaction of forming a tetrahydroxy-tetraamide-diurea.

### c) Preparation of a pigment dispersant with polyester tails

Into a 500 ml three-necked round-bottomed flask equipped with a reflux condenser, a mechanical stirrer, and a thermometer 40.21 grams (48.4 mmoles) of the tetrahydroxy-tetraamide-diurea of Example 1b were weighed together with 132.75 grams (0.861 mole) of hexahydrophthalic anhydride (ex Lonza) and 0.39 grams of a chromium based catalyst (Accelerator AMC-2, ex Flevo Chemie). The reaction mixture was heated to 155°C. Next 215.26 grams (0.878 mole) of Cardura® E10 (ex Resolution) were added over a period of 2.5 hours. An additional hour completed the reaction.

### Example 2: Preparation of a pigment dispersant with imidazolidinone groups

### a) Preparation of a hydroxy-amide-amine

Into a 500 ml three-necked round-bottomed flask equipped with a mechanical stirrer, a N₂-inlet, and a reflux condenser, 106.54 grams (1.033 moles) of diethylene triamine (ex Aldrich) and 20 grams of cyclohexane were weighed. The temperature was raised to 100°C (reflux) and next a mixture of 235.76 grams (2.065 moles) of ε-caprolactone (ex Aldrich) and 107.28 grams of t-amyl alcohol were added over a period of 2.5 hours. An extra two hours completed the reaction.

### b) Preparation of a tetrahydroxy-tetraamide-diurea block with additional imidazolidinone groups

Into a 250 ml three-necked round-bottomed flask equipped with a mechanical stirrer, a N₂-inlet, and a reflux condenser, 98.62 grams of the solution of the polyamine-derived compound of Example 2a were weighed together with 4.16 grams of K-Flex® XM-3323 (Ex King Industries), an amine-functional imidazolidinone. The temperature was raised to 80°C and next 26.3 grams (0.156 mole) of hexamethylene diisocyanate (ex Fluka) were added over a period of 30 minutes while keeping the reaction mixture at 80°C. The reaction was stopped 20 minutes after all isocyanate had been added.

### c) Preparation of a pigment dispersant with polyester tails

Into a 250 ml three-necked round-bottomed flask equipped with a reflux condenser, a mechanical stirrer, a N₂-inlet, and a thermometer 20.05 grams (theoretical: 68.7 mmoles of hydroxy groups) of the resin solution of Example 2b were weighed together with 53.05 grams (0.344 mole) of hexahydrophtalic anhydride (ex Lonza) and 0.17 grams of a Zn-based catalyst (Nuodex® ZN12 ex Sasol). The reaction mixture was heated to 145°C. Next, 77.04 grams (0.318 mole) of Cardura® E10 (ex Resolution) were added over a period of 2.5 hours. An additional hour at 150°C completed the reaction.

### Example 3: Preparation of a pigment dispersant with polyether tails

Into a 500 ml three-necked round-bottomed flask equipped with a mechanical stirrer, a N₂-inlet, and a reflux condenser, 9.82 grams of the resin of Example 2a, 50 grams of t-amyl alcohol, and 93.00 grams of Jeffamine® M1000 (ex Huntsman) were weighed. The reaction temperature was raised to 60°C and 10.40 grams (61.8 mmoles) of hexamethylene diisocyanate were added over a period of 30 minutes. After 15 minutes the temperature was raised to 80°C and vacuum was applied in order to remove solvents.

The product of the Examples showed very good pigment dispersant properties, had a desirable low viscosity, higher gloss, and was fully compatible with an acrylated resin, therefore no precipitate was formed, allowing the use of the formulation "as is", meaning that it does not require re-heating before use. It was found to be an improvement over conventional products, also because the polyamine derivative was easily dissolved in the resin, while it allowed for a much easier dispersion of the pigment into the formulation.

## Claims

1. A process wherein one or more polyamines, each with one or more -NH₂ functions and one or more second amine functions, said second amine functions having a lower lactone reactivity than said -NH₂ functions, is reacted in a first step with one or more lactones, hydroxyacids, cyclic carbonates, or mixtures thereof, to form a polyamine derived compound with amide and/or urethane groups, which polyamine-derived compound is reacted in a second step with one or more at least bifunctional amine-specific reagents to form an intermediate optionally comprising ester and/or carbonate groups, wherein in the second step optionally an additional amine modifier of formula III)
(Y)ₓ(Y")_{y}(Y')_{z}NH_{3-x-y-z} III)
wherein x is an integer of 0, 1 or 2, y is an integer of 0, 1 or 2, z is an integer of 0 or 1, wherein x+y is 1 or 2, x+y+z is 1 or 2, Y represents an (anchoring) moiety with affinity for a pigment surface or substrate, Y" represents a (stabilising) moiety with affinity for the matrix, and Y' represents a further group that is neither an anchoring moiety nor a stabilising moiety, is co-reacted
and in the intermediate at least two polyamine residues, or if a modifier is co-reacted, at least one polyamine residue and at least one optional amine modifier residue, are linked by the bifunctional amine-specific reagent.

2. A process according to claim 1 wherein in the second step an intermediate comprising at least two polyamine residues is formed.

3. A process according to claim 1 or 2 wherein the number of lactone, hydroxy acid and/or cyclic carbonate molecules is from 0.1 to 10 times the number of -NH₂ groups of the polyamine.

4. A process according to any one of claims 1 to 3 wherein the bifunctional amine-specific reagent is used in an amount such that the number of amine-reactive groups corresponds to from 0.1 to 10 times the sum of the number of second amine functions of the polyamine-derived compound and the number of amine functions of the optional amine modifier.

5. A process according to any one of the preceding claims 1-4 wherein a polyamine is used of formula I) 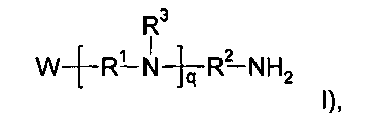 wherein q is an integer from 1 to 10, wherein R¹ and R², independently, are selected from alkylene groups with from 1 to 10 carbon atoms, wherein each of R³, independently, is selected from the group consisting of hydrogen, hydroxyalkyls, alkylamines, polyalkylamines, and polyalkylpolyamines, and wherein W is hydroxy or amine.

6. A process according to any one of the preceding claims which comprises a further step wherein one or more of the -OH groups which are present after the first step are reacted to attach a matrix-compatible moiety with a molecular weight of more than 250 to said intermediate, with said further step being conducted either between the first and second steps or, preferably, after the second step.

7. A process according to claim 6 wherein the -OH groups are reacted with one or more compounds selected from the group consisting of epoxides, lactones, cyclic carbonates, hydroxy acids, and other suitable conventional reactants to form polyesters, to form matrix-compatible linear or branched, substituted or unsubstituted, preferably unsubstituted, C₄-C₃₀ alkyl, polyester, polyether, polyetherester or polyesterether groups.

8. A process according to any one of the preceding claims wherein at least one amine modifier of formula III is co-reacted in the second step.

9. Polyamine derivative of formula II : 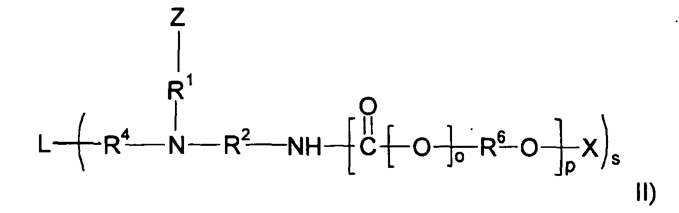 wherein each R⁴NR¹ZR²NH moiety is a residue of a polyamine, each C(O)[O]R⁶O moiety is a residue of a lactone, hydroxyacid and/or cyclic carbonate, L is a residue of at least bifunctional amine-specific reagent, R¹ and R² are as defined above for formula I), each index o will independently be 0 or 1, index p represents the average number of moiety C(O)[O]R⁶O per R⁴NR¹ZR²NH moiety and has a value ranging from 0.1 to 30, each X is hydrogen or, wholly or partly, a substituted or unsubstituted, linear or branched, hydrocarbon group, polyester, polyether, polyetherester or polyesterether group, index s represents an integer of 1 to 10, wherein if s is 1, the amine specific reagent L is further reacted with a compound of formula III as defined above, R⁴ represents a group R³ minus a proton, R³ is as defined above for formula I), Z- presents a group W'-[R¹-NR⁵]_{q-1}-, wherein W' is W as defined for formula I above or the reaction product of group W with at least one lactone, hydroxyacid and/or cyclic carbonate and each R⁵ independently is a group R³ or the reaction product of R³ with amine-specific reagent L.

10. Polyamine derivative obtainable by a process according to any one of the preceding claims 1-8.

11. Use of the polyamine derivative of claim 9 or 10 in printing ink formulations or coating compositions.

12. Use of the polyamine derivative of claim 9 or 10 as a pigment dispersant.

## Patentansprüche

1. Verfahren, wobei ein oder mehrere Polyamine, welche jeweils eine oder mehrere NH₂₋Funktionen und eine oder mehrere Sekundäraminfunktionen aufweisen, wobei die Sekundäraminfunktionen eine geringere Lactonreaktivität als die NH₂-Funktionen aufweisen, in einem ersten Schritt mit einem/r oder mehreren Lactonen, Hydroxysäuren, cyclischen Carbonaten oder Gemischen davon umgesetzt werden, um eine von dem Polyamin abgeleitete Verbindung mit Amid- und/oder Urethangruppen herzustellen, wobei die von dem Polyamin abgeleitete Verbindung in einem zweiten Schritt mit einem oder mehreren zumindest bifunktionellen, aminspezifischen Reagenzien umgesetzt wird, um ein Zwischenprodukt herzustellen, welches gegebenenfalls Ester- und/oder Carbonatreste umfasst, wobei im zweiten Schritt gegebenenfalls ein zusätzlicher Aminmodifizierer der Formel III)
(Y)ₓ(Y")_{y}(Y')_{z}NH_{3-x-y-z} III)
wobei x eine ganze Zahl von 0, 1 oder 2 ist, y eine ganze Zahl von 0, 1 oder 2 ist, z eine ganze Zahl von 0 oder 1 ist, wobei x + y gleich 1 oder 2 ist, x + y + z gleich 1 oder 2 ist, Y eine (verankernde) Einheit mit einer Affinität für eine Pigmentoberfläche oder -Substrat darstellt, Y" eine (stabilisierende) Einheit mit einer Affinität für die Matrix darstellt und Y' einen weiteren Rest darstellt, welcher weder eine verankernde Einheit noch eine stabilisierende Einheit ist, mit umgesetzt wird und in dem Zwischenprodukt mindestens zwei Polyaminreste, oder wenn ein Modifizierer mit umgesetzt wird, mindestens ein Polyaminrest und mindestens ein optionaler Aminmodifizier-Rest, durch das bifunktionelle, aminspezifische Reagenz verbunden sind.

2. Verfahren gemäß Anspruch 1, wobei im zweiten Schritt ein Zwischenprodukt gebildet wird, welches mindestens zwei Polyaminreste umfasst.

3. Verfahren gemäß Anspruch 1 oder 2, wobei die Anzahl an Lacton-, Hydroxysäure- und/oder cyclischen Carbonatmolekülen das 0,1- bis 10fache der Anzahl der NH-Gruppen des Polyamins ist.

4. Verfahren gemäß einem der Ansprüche 1 bis 3, wobei das bifunktionelle, aminspezifische Reagenz in einer solchen Menge verwendet wird, dass die Anzahl der aminreaktiven Gruppen 0,1- bis 10fach der Summe der Anzahl der Sekundäraminfunktionen der von dem Polyamin abgeleiteten Verbindung und der Anzahl der Aminfunktionen des optionalen Aminmodifizierers entspricht.

5. Verfahren gemäß einem der vorstehenden Ansprüche 1 bis 4, wobei ein Polyamin der Formel I) 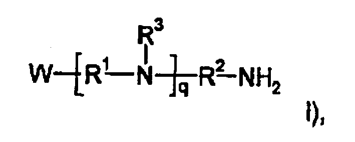 verwendet wird,
wobei q eine ganze Zahl von 1 bis 10 ist, wobei R¹ und R² unabhängig voneinander aus Alkylenresten mit 1 bis 10 Kohlenstoffatomen ausgewählt sind, wobei R³ jeweils unabhängig aus Wasserstoff, Hydroxyalkylen, Alkylaminen, Polyalkylaminen und Polyalkylpolyaminen, ausgewählt ist und wobei W Hydroxy oder Amin ist.

6. Verfahren gemäß einem der vorstehenden Ansprüche, welches einen weiteren Schritt umfasst, wobei eine oder mehrere der OH-Gruppen, welche nach dem ersten Schritt vorhanden sind, umgesetzt werden, um eine Matrix-kompatible Einheit mit einem Molekulargewicht von mehr als 250 an das Zwischenprodukt zu binden, wobei der weitere Schritt entweder zwischen dem ersten und dem zweiten Schritt oder vorzugsweise nach dem zweiten Schritt ausgeführt wird.

7. Verfahren gemäß Anspruch 6, wobei die OH-Gruppen mit einer oder mehreren Verbindungen umgesetzt werden, welche aus Epoxiden, Lactonen, cyclischen Carbonaten, Hydroxysäuren und anderen geeigneten üblichen Reaktanten, um Polyester zu bilden, ausgewählt sind um Matrix-kompatible, lineare oder verzweigte, substituierte oder unsubstituierte, vorzugsweise unsubstituierte, C₄-C₃₀-Alkyl-, Polyester-, Polyether-, Polyetherester- oder Polyesteretherreste zu bilden.

8. Verfahren gemäß einem der vorstehenden Ansprüche, wobei mindestens ein Aminmodifizierer der Formel III im zweiten Schritt mit umgesetzt wird.

9. Polyaminderivat der Formel II: 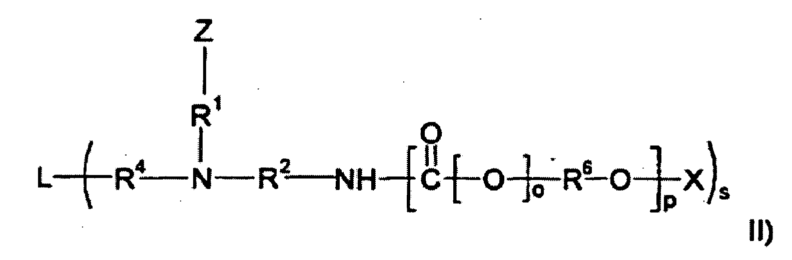 wobei die R⁴NR¹ZR²NH-Einheit jeweils ein Rest eines Polyamins ist, die C(O)[O]R⁶O-Einheit jeweils ein Rest eines Lactons, einer Hydroxysäure und/oder eines cyclischen Carbonats ist, L ein Rest eines mindestens bifunktionellen, aminspezifischen Reagenzes ist, R¹ und R² wie vorstehend für Formel I) definiert sind, der Index o jeweils unabhängig 0 oder 1 ist, der Index p die durchschnittliche Anzahl an C(O)[O]R⁶O-Einheiten pro R⁴NR¹ZR²NH-Einheit darstellt und einen Wert im Bereich von 0,1 bis 30 aufweist, X jeweils Wasserstoff oder, ganz oder teilweise, ein substituierter oder unsubstituierter, linearer oder verzweigter Kohlenwasserstoff-, Polyester-, Polyether-, Polyetherester- oder Polyesteretherrest ist, der Index s eine ganze Zahl von 1 bis 10 darstellt, wobei wenn s gleich 1 ist, das aminspezifische Reagens L ferner mit einer Verbindung der Formel III wie vorstehend definiert umgesetzt ist, R⁴ einen Rest R³ minus einem Proton darstellt, R³ wie vorstehend für Formel I) definiert ist, Z- einen Rest W'-[R¹-NR⁵]_{q-1}- darstellt, wobei W' gleich W wie vorstehend für Formel I definiert ist oder das Reaktionsprodukt des Restes W mit mindestens einem Lacton, einer Hydroxysäure und/oder einem cyclischen Carbonat ist und R⁵ jeweils unabhängig ein Rest R³ oder das Reaktionsprodukt von R³ mit dem aminspezifischen Reagens L ist.

10. Polyaminderivat, erhältlich durch ein Verfahren gemäß einem der vorstehenden Ansprüche 1 bis 8.

11. Verwendung des Polyaminderivats gemäß Anspruch 9 oder 10 in Druckfarbenformulierungen oder Beschichtungszusammensetzungen.

12. Verwendung des Polyaminderivats gemäß Anspruch 9 oder 10 als ein Pigmentdispersionsmittel.

## Revendications

1. Procédé dans lequel on fait réagir une ou plusieurs polyamines, ayant chacune une ou plusieurs fonctions -NH₂ et une ou plusieurs secondes fonctions amine, lesdites secondes fonctions amine ayant une réactivité à la lactone inférieure auxdites fonctions -NH₂, dans une première étape, avec un ou plusieurs composés choisis parmi des lactones, des hydroxyacides, des carbonates cycliques ou leurs mélanges, pour former un composé dérivé de polyamine avec des groupes amide et/ou uréthane, lequel composé dérivé de polyamine réagit dans une seconde étape avec un ou plusieurs réactifs spécifiques à l'amine au moins bifonctionnels pour former un intermédiaire comprenant éventuellement des groupes ester et/ou carbonate, seconde étape dans laquelle on fait co-réagir éventuellement un agent modifiant de type amine supplémentaire de formule (III)
(Y)ₓ(Y")_{y}(Y')_{z}NH_{3-x-y-z} (III)
dans laquelle x représente un nombre entier valant 0, 1 ou 2, y représente un nombre entier valant 0, 1 ou 2, z représente un nombre entier valant 0 ou 1, la somme x + y vaut 1 ou 2, la somme x + y + z vaut 1 ou 2, Y représente un fragment (d'ancrage) avec une affinité pour la surface d'un pigment ou un substrat, Y" représente un fragment (stabilisant) avec une affinité pour la matrice et Y' représente un groupe supplémentaire qui est ni un fragment d'ancrage ni un fragment stabilisant, et dans l'intermédiaire au moins deux résidus polyamine, ou si un agent modifiant co-réagit, au moins un résidu de polyamine et au moins un résidu d'agent modifiant de type amine facultatif, sont liés par le réactif spécifique à l'amine bifonctionnel.

2. Procédé selon la revendication 1, dans lequel dans la seconde étape, il se forme un intermédiaire comprenant au moins deux résidus polyamine.

3. Procédé selon la revendication 1 ou 2, dans lequel le nombre de molécules de lactone, d'hydroxyacide et/ou de carbonate cyclique est de 0,1 à 10 fois le nombre de groupes -NH₂ de la polyamine.

4. Procédé selon l'une quelconque des revendications 1 à 3, dans lequel le réactif spécifique à l'amine bifonctionnel est utilisé en une quantité telle que le nombre de groupes réactifs à l'amine correspond de 0,1 à 10 fois à la somme du nombre des secondes fonctions amine du composé dérivé de polyamine et du nombre de fonctions amine de l'agent modifiant de type amine facultatif.

5. Procédé selon l'une quelconque des revendications 1 à 4 précédentes, dans lequel on utilise une polyamine de formule (I) : 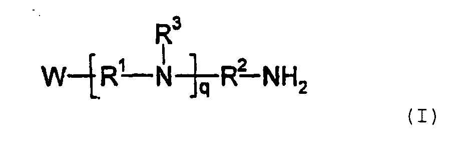 dans laquelle q représente un nombre entier valant de 1 à 10, R¹ et R², sont indépendamment choisis parmi des groupes alkylène ayant de 1 à 10 atomes de carbone, chaque R³, est indépendamment choisi dans l'ensemble comprenant l'atome d'hydrogène et les groupes hydroxyalkyle, alkylamine, polyalkylamine et polyalkylpolyamine, et W représente un groupe hydroxy ou amine.

6. Procédé selon l'une quelconque des revendications précédentes, qui comprend une étape supplémentaire dans laquelle un ou plusieurs des groupes -OH qui sont présents après la première étape réagissent pour fixer un fragment compatible avec la matrice ayant une masse moléculaire supérieure à 250 audit intermédiaire, ladite étape supplémentaire étant menée soit entre les première et seconde étapes ou de préférence après la seconde étape.

7. Procédé selon la revendication 6, dans lequel les groupes -OH réagissent avec un ou plusieurs composés choisis dans l'ensemble comprenant les époxydes, les lactones, les carbonates cycliques, les hydroxyacides et d'autres corps réactionnels classiques convenables pour former des polyesters, pour former des groupes alkyle en C₄-C₃₀ linéaires ou ramifiés, substitués ou non substitués, de préférence non substitués, polyester, polyéther, polyétherester ou polyesteréther compatibles avec la matrice.

8. Procédé selon l'une quelconque des revendications précédentes, dans lequel on fait co-réagir dans la seconde étape, au moins un agent modifiant de type amine de formule (III).

9. Dérivé de polyamine de formule (II) : 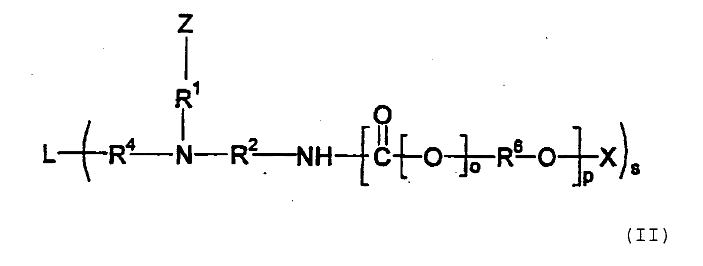 dans laquelle chaque fragment R⁴NR¹ZR²NH est un résidu d'une polyamine, chaque fragment C(O) [O]R⁶O est un résidu d'une lactone, d'un hydroxyacide et/ou d'un carbonate cyclique, L est un résidu d'un réactif spécifique à l'amine au moins bifonctionnel, R¹ et R² sont tels que définis ci-dessus pour la formule (I), chaque indice o aura indépendamment une valeur de 0 ou 1, l'indice p représente le nombre moyen de fragments C (O) [O] R⁶O par fragment R⁴NR¹ZR²NH et possède une valeur se situant de 0,1 à 30, chaque X représente un atome d'hydrogène ou, totalement ou partiellement, un groupe hydrocarboné substitué ou non substitué, linéaire ou ramifié, un groupe polyester, polyéther, polyétherester ou polyesteréther, l'indice s représente un nombre entier valant de 1 à 10 où si s vaut 1, le réactif spécifique à l'amine L réagit encore avec un composé de formule III tel que défini ci-dessus, R⁴ représente un groupe R³ moins un proton, R³ est tel que défini ci-dessus pour la formule I, Z- représente un groupe W' - [R¹-NR⁵] _{q-1-}, où W' est W tel que défini pour la formule I ci-dessus ou le produit de réaction du groupe W avec au moins une lactone, un hydroxyacide et/ou un carbonate cyclique, et chaque R⁵ représente indépendamment un groupe R³ ou le produit de réaction de R³ avec un réactif spécifique à l'amine L.

10. Dérivé de polyamine que l'on obtient grâce au procédé selon l'une quelconque des revendications précédentes 1 à 8.

11. Utilisation du dérivé de polyamine selon la revendication 9 ou 10, dans des formulations d'encres d'impression ou des compositions de revêtements.

12. Utilisation du dérivé de polyamine selon la revendication 9 ou 10, comme dispersant de pigment.
